# EUROPEAN PATENT SPECIFICATION

(11) **EP 3 272 376 B1**
(45) Date of publication and mention of the grant of the patent: **08.12.2021**
(21) Application number: 16768837.3
(22) Date of filing: 23.03.2016
(51) Int. Cl.: A61M 1/36, A61M 1/34

(54) **MIXING CHAMBER**
MISCHKAMMER
CHAMBRE DE MÉLANGE

(30) Priority: 23.03.2015 JP 2015059877
(43) Date of publication of application: 24.01.2018
(73) Proprietor: Nipro Corporation, Osaka-shi, Osaka 531-8510 (JP)
(72) Inventor: HIDAKA, Masato, Osaka-shi, Osaka 531-8510 (JP); ISHIKURA, Kohzo, Osaka-shi, Osaka 531-8510 (JP); KUDO, Tatsuya, Osaka-shi, Osaka 531-8510 (JP); YAMAGUCHI, Takeshi, Osaka-shi, Osaka 531-8510 (JP)
(74) Representative: Prüfer & Partner mbB Patentanwälte · Rechtsanwälte
(86) International application number: PCT/JP2016/059255
(87) International publication number: WO 2016/152934

(56) References cited:
- JP-A- 2005 530 543
- JP-A- 2012 095 842
- JP-A- 2012 095 842
- JP-A- 2015 085 181
- JP-A- 2015 085 181
- US-A1- 2005 247 203

## Description

### TECHNICAL FIELD

The present invention relates to a mixing chamber provided on a blood circuit.

### BACKGROUND ART

Conventionally, a chamber has been disclosed in Japanese Patent Laying-Open No. 2012-95841 (PTD 1), for example.

### CITATION LIST

### PATENT DOCUMENT

PTD 1: Japanese Patent Laying-Open No. 2012-95841
PTD 2: US Patent No. 5503801
PTD 3: European Patent Publication No. 0728509
PTD 4: European Patent Publication No. 2055331
PTD 5: WO 2004/000391 A1
PTD 6: JP 2012 095842 A

In particular, PTD 5 discloses a gas separation device for a physiological fluid, comprising a containing body having at least a first inlet aperture for a physiological fluid, positioned with a tangential direction of access, at least one outlet aperture for the said fluid, spaced apart from the said inlet aperture, and a guide element housed within the said body. The guide element has a continuous active surface designed to contact and guide the said fluid and delimits, together with the containing body, a first annular chamber into which the first inlet aperture opens directly.

PTD 6 discloses an air trap chamber allowing priming of a blood purifier to be efficiently performed in a simple method.

### SUMMARY OF INVENTION

### TECHNICAL PROBLEM

Online HDF (Hemo Dialysis Filtration) may be performed to mix the blood and a dialysate (displacement liquid). A conventional chamber has had a problem of occurrence of bubbling when a displacement liquid is introduced into the blood in the chamber. Accordingly, an object of the present invention is to provide a mixing chamber that can solve the above-mentioned problem.

### SOLUTION TO PROBLEM

A mixing chamber according to the present invention is a mixing chamber according to claim 1.

In the mixing chamber configured as described above, a liquid level of the liquid in the chamber can be set at a position higher than the first inlet and the second inlet in a use state of the mixing chamber. Therefore, when the liquid is introduced, the liquid can be stored in the space located above the first inlet, and suction of the gas by the first inlet can be suppressed. As a result, inflow of the air into the first inlet and a line connected thereto can be suppressed. In addition, the liquid can be introduced without being dropped onto the liquid level. Therefore, bubbling can be prevented.

The mixing chamber is configured such that the liquid introduced from the first inlet into the main body portion moves toward a direction displaced from a center of the main body portion.

The inner circumferential surface of the main body portion guides, in a swirling manner, the liquid flowing from the first inlet into the main body portion. The inner circumferential surface of the main body portion guides, in a swirling manner, the liquid flowing from the second inlet into the main body portion.

Preferably, a curved surface portion configured to guide the liquid in a rotation direction is formed on an inner surface of the main body portion in a flow direction of the liquid introduced from the first inlet into the main body portion.

Preferably, the first inlet extends along an inner circumferential surface of one side surface of the main body portion.

Preferably, the second inlet extends along the inner circumferential surface of the one side surface of the main body portion.

Preferably, the inner circumferential surface of the main body portion from the first inlet to the second inlet has a substantially circular cylindrical shape.

Preferably, the inner circumferential surface of the main body portion has a curved surface, and at least one of the first inlet and the second inlet extends along a direction of a tangent to the curved surface.

Preferably, the main body portion extends in a longitudinal direction, the main body portion has a first port extending to be substantially orthogonal to the longitudinal direction, one end of the first port is connected to the first inlet, and the other end of the first port is connected to a displacement liquid line.

Preferably, the main body portion extends in the longitudinal direction, the main body portion has a second port extending to be substantially orthogonal to the longitudinal direction, one end of the second port is connected to the second inlet, and the other end of the second port is connected to a blood line.

A distance from a bottom of the main body portion to the second port is not shorter than 40 mm.

Preferably, a distance between the first port and the second port is not shorter than 0.5 cm.

Preferably, a groove connecting to at least one of the first port and the second port is provided in the side surface of the main body portion.

Preferably, a filter configured to filter a mixed liquid of the liquid and the blood is provided below the first and second ports in the main body portion, and the filter has a cylindrical shape.

Preferably, a volume of the space is not smaller than 0.5 cm³.

Preferably, a volume of the space is not smaller than 3 to 9 cm³ and an inner diameter of the space is 10 to 35 mm.

Preferably, a flow resistance of a mixed liquid is substantially equal at respective height surfaces in the main body portion.

Preferably, a transversely blocking wall member that interferes with a vortex flow is not provided in the main body portion. In this case, staying of the mixed liquid can be prevented. Furthermore, an abnormal flow such as a turbulent flow and a backflow is less likely to occur and damage to the blood components can be reduced.

Preferably, the main body portion includes an upper member that is provided with the first inlet and the second inlet, and a circular cylindrical lower member that is not provided with the first inlet and the second inlet and is in contact with the upper member.

### BRIEF DESCRIPTION OF DRAWINGS

Fig. 1 is a schematic view of a blood circuit on which a mixing chamber according to an embodiment is used.
Fig. 2 is a front view of the mixing chamber according to a first embodiment.
Fig. 3 is a perspective view of the mixing chamber according to the first embodiment.
Fig. 4 is a front view of the mixing chamber according to the first embodiment.
Fig. 5 is a plan view of the mixing chamber according to the first embodiment.
Fig. 6 is a cross-sectional view taken along line VI-VI in Fig. 5.
Fig. 7 is a cross-sectional view taken along line VII-VII in Fig. 5.
Fig. 8 is a cross-sectional view taken along line VIII-VIII in Fig. 5.
Fig. 9 is an enlarged front view of a main body portion of the mixing chamber.
Fig. 10 is a cross-sectional view taken along line X-X in Fig. 9.
Fig. 11 is a cross-sectional view taken along line XI-XI in Fig. 9.
Fig. 12 is a perspective view of the main body portion of the mixing chamber.
Fig. 13 is a perspective view of the main body portion of the mixing chamber having two ports at the top.
Fig. 14 is a perspective view of the main body portion of the mixing chamber having one port at the top.
Fig. 15 is a photograph of the mixing chamber.

### DESCRIPTION OF EMBODIMENTS

Embodiments of the present invention have been described hereinafter with reference to the drawings. In the following embodiments, the same or corresponding portions are denoted by the same reference characters and description thereof will not be repeated. Combining the embodiments is also possible.

### (First Embodiment)

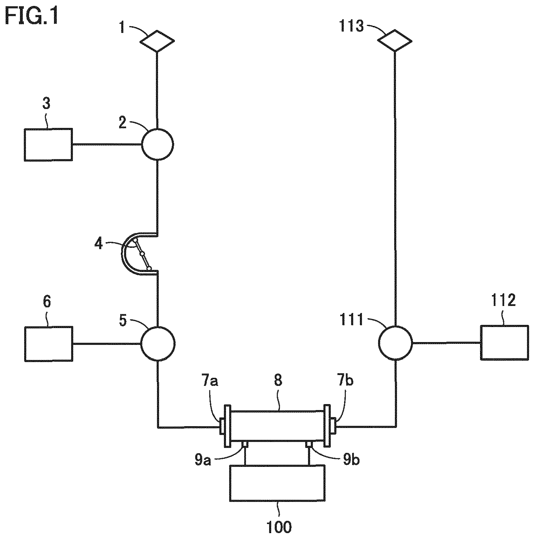

Referring to Fig. 1, a blood circuit has a blood introduction port 1 through which the blood is taken out from a patient, a blood removal pressure measurement site 2 connected to blood introduction port 1, a pressure transducer 3 configured to measure a blood pressure of blood removal pressure measurement site 2, a blood pump 4 configured to pressurize the blood discharged from blood removal pressure measurement site 2, a PD pressure measurement site 5 located on the downstream side of blood pump 4, a pressure transducer 6 configured to measure a blood pressure of PD (Pre-dialyzer) pressure measurement site 5, a dialyzer 8 configured to accept the blood discharged from PD pressure measurement site 5 through a dialyzer blood introduction port 7a, a venous pressure measurement site 111 located on the downstream side of a dialyzer blood delivery port 7b of dialyzer 8, a pressure transducer 112 configured to measure a blood pressure of venous pressure measurement site 111, and a blood delivery port 113 through which the blood is returned to the patient.

Dialyzer 8 is connected to a dialysis apparatus main body 100 via a dialysate delivery port 9a and a dialysate introduction port 9b. Dialyzer 8 uses a dialysate to remove waste products in the blood and adjust the water in the blood.

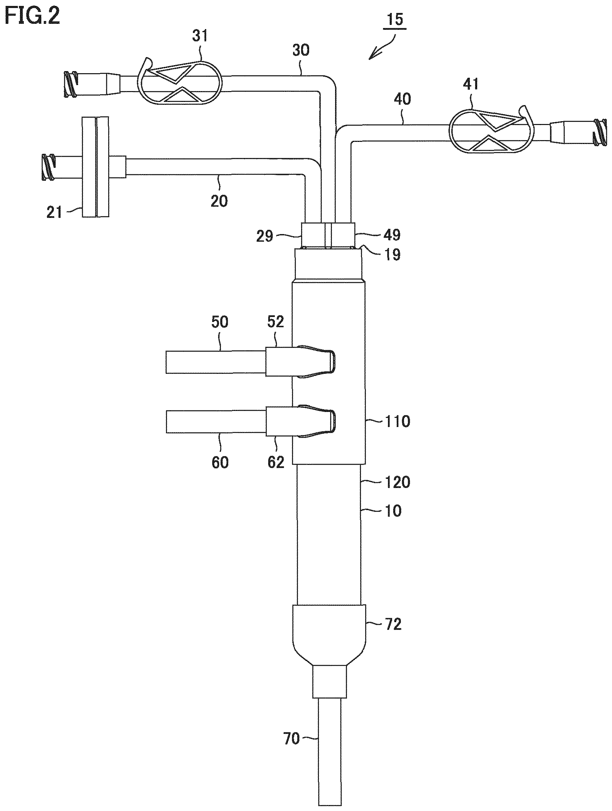

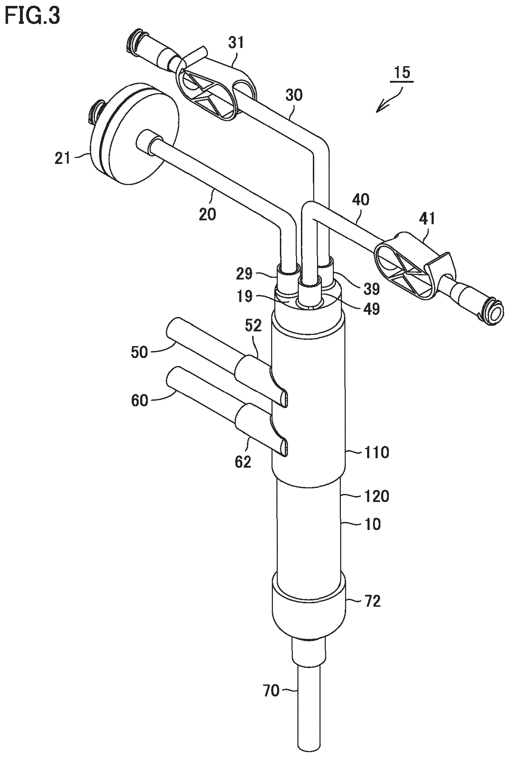

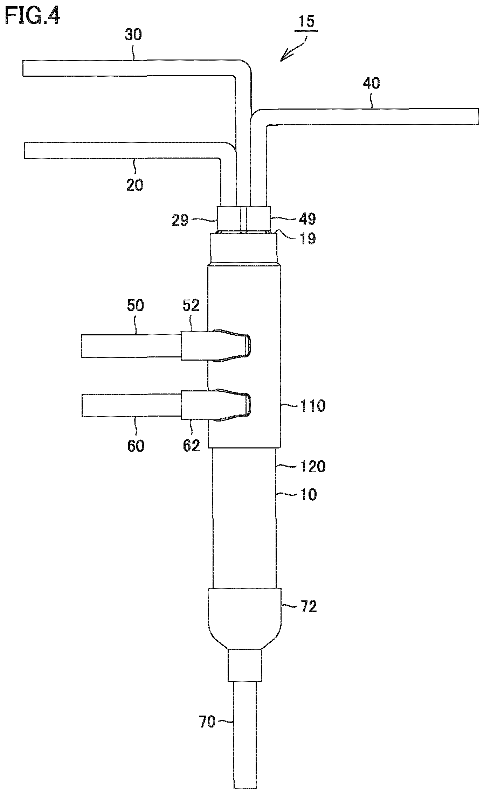

Referring to Figs. 2 to 4, a mixing chamber 15 includes a main body portion 10, and ports 29, 39, 49, 52, 62, and 72 connected to main body portion 10.

A pressure measurement line 20 is connected to port 29. Pressure measurement line 20 is provided with a transducer protection filter 21.

A liquid level adjustment line 40 is connected to port 49. Liquid level adjustment line 40 is provided with a clip 41. Liquid level adjustment line 40 can be closed by clip 41.

A medical agent injection line 30 configured to inject a small amount of medical agent is connected to port 39. Medical agent injection line 30 is provided with a clip 31. Medical agent injection line 30 can be closed by clip 31.

Main body portion 10 is formed by assembling an upper housing and a lower housing. A displacement liquid line 50 is connected to port 52. A blood line 60 is connected to port 62. A mixed liquid line 70 is connected to port 72. Port 52 and port 62 are formed on the upper housing, and thus, port 52 and port 62 are designed such that there is no level difference therebetween and smooth mixing is achieved without bubbling. In addition, mixed liquid line 70 is provided at the bottom of the lower housing and a closed-end cylindrical filter is arranged to surround mixed liquid line 70.

In offline HDF and online HDF (Hemo Dialysis Filtration), it is necessary to mix the blood and a liquid (normal saline or dialysate). HDF refers to treatment in which blood dialysis and filtration are performed simultaneously. HDF allows balanced removal of various waste products from a waste product of low molecular weight to a waste product of relatively high molecular weight.

Since a large amount of water is lost during filtration, it is necessary to add a dialysate (displacement liquid) as a replacement liquid. A method for adding and diluting the dialysate includes a pre-dilution method and a post-dilution method. In the pre-dilution method, the displacement liquid is added to the blood to dilute the blood before a dialyzer step. The diluted blood is filtered by a dialyzer. In the post-dilution method, the blood is filtered by the dialyzer, and thereafter, the dialysate is added.

In the pre-dilution method, the blood before entering dialyzer 8 is introduced into blood line 60. In the post-dilution method, the blood having exited from dialyzer 8 is introduced into blood line 60. The displacement liquid is introduced from dialysis apparatus main body 100 into displacement liquid line 50. The displacement liquid includes a priming liquid. Specifically, the priming liquid (normal saline) serving as the displacement liquid is introduced from displacement liquid line 50 during priming.

In the present embodiment, displacement liquid line 50 is connected to the upper side of main body portion 10 and blood line 60 is connected to the lower side of main body portion 10. However, blood line 60 may be connected to the upper side of main body portion 10 and blood line 60 may be connected to the lower side of main body portion 10.

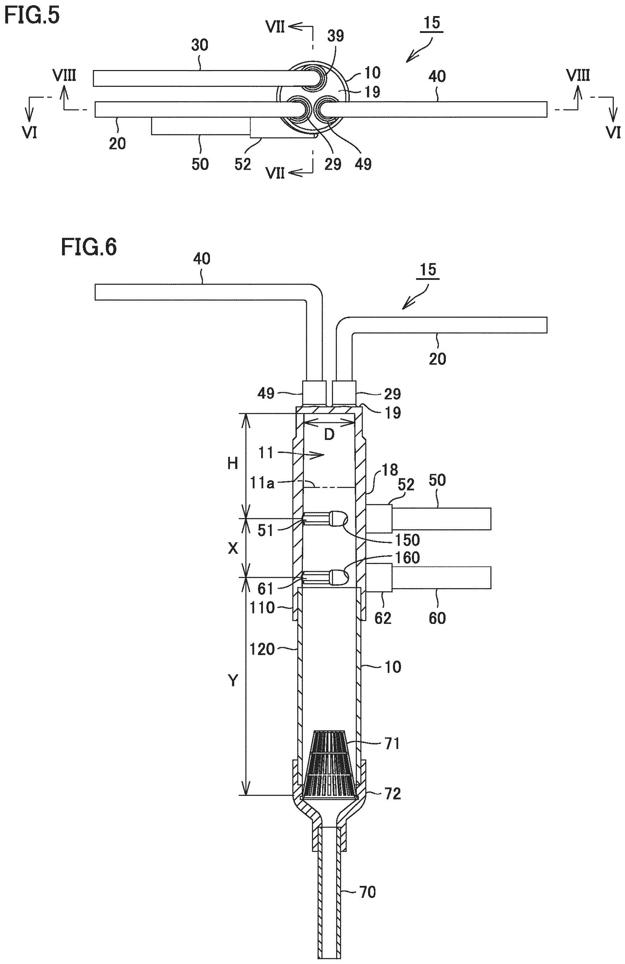

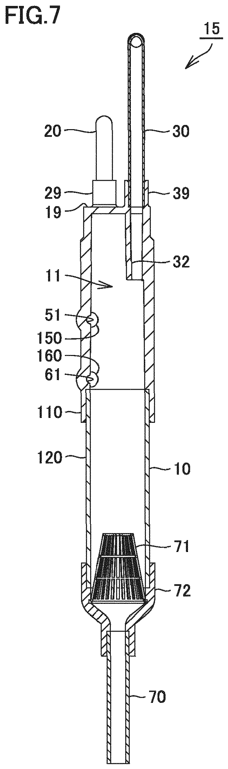

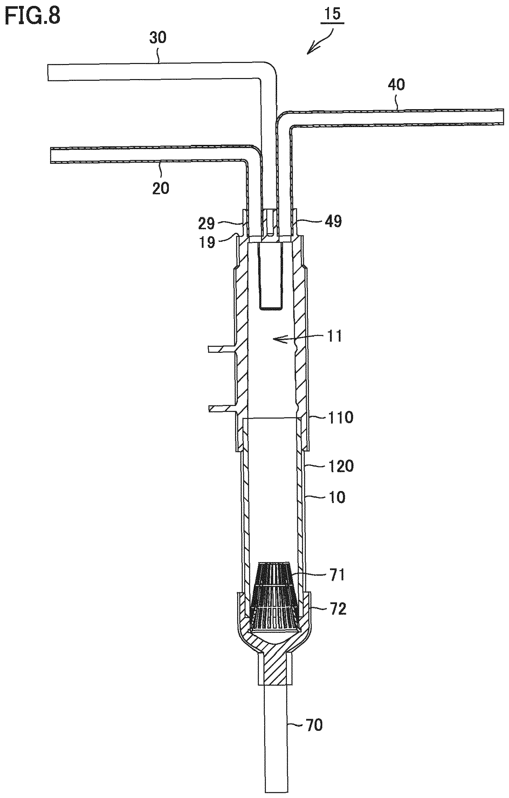

Referring to Figs. 5 to 8, grooves 51 and 61 are provided in main body portion 10 having a circular cylindrical shape. Grooves 51 and 61 extend in the shape of an arc along an inner circumferential surface of main body portion 10. Grooves 51 and 61 extend parallel to each other. An inner surface of main body portion 10 does not necessarily need to have a circular cylindrical shape (also including an elliptic cylindrical shape), and may have, for example, a rectangular cylindrical shape having a rectangular cross section. The inner surface of main body portion 10 having a substantially circular cylindrical shape, not a rectangular cylindrical shape, can make a vortex flow more likely to occur and make staying less likely to occur. When the inner surface of main body portion 10 has a rectangular cylindrical shape, a polygonal shape having six or more vertexes is preferable, and a regular polygonal shape is more preferable, in order to bring the shape of the inner surface of main body portion 10 closer to the circular cylindrical shape. The inner surface of main body portion 10 preferably has a curved surface, and the curved surface formed on the inner surface of main body portion 10 located at the same height as ports 52 and 62 particularly makes a vortex flow more likely to occur. The optimum cylindrical shape is a circular cylindrical shape having a cross section of a true circle. Even if the inner surface of main body portion 10 near mixed liquid line 70 has a rectangular cylindrical shape, this shape has not so much influence on ease of formation of thrombus. However, by making the shape of the inner surface of main body portion 10 near mixed liquid line 70 substantially identical to the shape of the inner surface of main body portion 10 near ports 52 and 62, the mixing property of the blood and the displacement liquid can become more uniform. An inner side surface of main body portion 10 is linearly formed in the cross section in Fig. 6. As a result, a flow of a mixed liquid of the blood and the displacement liquid is not blocked on the side surface. In addition, the inner surface of main body portion 10 located at the same height as ports 52 and 62 does not have a protrusion and the like that interfere with a vortex flow, and thus, the flow of the blood and the displacement liquid is not blocked.

The depth of grooves 51 and 61 may be constant. The depth of grooves 51 and 61 may become shallower with increasing distance from ports 52 and 62. Grooves 51 and 61 extend to be substantially orthogonal to the longitudinal direction of main body portion 10. Bottom surfaces of grooves 51 and 61 are connected to the inner surface of the housing without any level difference.

An area located above port 52 corresponds to an upper space 11. The air is mainly stored in upper space 11. Since ports 52 and 62 are provided on the side surface of main body portion 10 and the upper space is located above port 52, the liquid level of the blood in main body portion 10 can be made higher than port 52, and bubbling can become less likely to occur when the displacement liquid introduced from port 52 and the blood introduced from port 62 are introduced into main body portion 10. In addition, the first port through which the displacement liquid is introduced is provided above the second port through which the blood is introduced. Therefore, a layer of water is likely to be formed above the blood, and thus, formation of thrombus caused by contact between the blood and the air can become less likely.

When a first inlet 150 is located at the center of main body portion 10 (at the center of a cross section orthogonal to the longitudinal direction of main body portion 10) (e.g., European Patent Publication No. 2055331), the liquid introduced from first inlet 150 flows toward the center of main body portion 10. As a result, the flow can hit the opposite walls, which can cause a turbulent flow. When the turbulent flow occurs, it becomes difficult to predict the flow and the risk of occurrence of an unintended upward flow increases. In order to prevent the occurrence of such a situation, one end of port 52 is connected to first inlet 150 formed to extend along the inner circumferential surface of one side surface of main body portion 10 in the present embodiment. The other end of port 52 is connected to displacement liquid line 50 formed by a tube. One end of port 62 is connected to a second inlet 160. The other end of port 62 is connected to blood line 60 formed by a tube. First inlet 150 and second inlet 160 are provided to extend in a direction of a tangent to the circular cylindrical inner circumferential surface of main body portion 10. First inlet 150 and second inlet 160 extend along the inner circumferential surface of one side surface of main body portion 10. In this case, the liquids introduced from first inlet 150 and second inlet 160 face in a direction displaced from the center of main body portion 10, and thus, the liquids introduced from first inlet 150 and second inlet 160 are introduced toward the direction displaced from the center of main body portion 10. Furthermore, first inlet 150 and second inlet 160 face toward a C-shaped curved line portion of main body portion 10. Due to the presence of the C-shaped curved surface portion in the flow direction of the liquids introduced from first inlet 150 and second inlet 160, the liquids introduced from first inlet 150 and second inlet 160 are guided in one direction (rotation direction) along the C-shaped curved surface. Therefore, the liquids flowing from first inlet 150 and second inlet 160 into main body portion 10 have a vortex-like flow. Ports 52 and 62 are arranged to be substantially orthogonal to the longitudinal direction of main body portion 10.

The suitable inner diameter of first inlet 150 is φ2 to 5 mm. If the inner diameter is too small, the momentum of the flow can become too strong and bubbling can occur, because the liquid may, in some cases, be flown at a high flow rate. Therefore, the inner diameter of not smaller than 2 mm is preferable. If the inner diameter is too large, the momentum of the flow becomes weak. Although the flow rate varies depending on a treatment method, the flow rate is approximately 150 to 300 cm³/min in the case of the pre-dilution method, and is approximately 20 to 80 cm³/min in the case of the post-dilution method. The above-described inner diameter is suitable for the pre-dilution method.

First inlet 150 and second inlet 160 are formed in the direction of the tangent to the circular cylinder inside main body portion 10 having a substantially circular cylindrical shape. Therefore, a layer of blood is likely to be formed axially in the central portion. However, this layer is a layer formed as a result of mixing, and thus, the blood concentration is low and the risk of thrombus is low.

When the displacement liquid and the blood are introduced from each inlet into main body portion 10, the displacement liquid and the blood flow along the inner side surface of main body portion 10 and a vortex flow is formed at the center of main body portion 10. When the flow rate of each of the displacement liquid and the blood is increased, the liquid level of the dialysate in main body portion 10 forms a shape having a recessed central portion.

The blood mixed with the displacement liquid flows inside main body portion 10 as a spiral flow. Due to the spiral flow, a flow path of the mixed liquid becomes longer, and thus, the displacement liquid and the blood can be sufficiently mixed. Furthermore, when the momentum of the spiral flow is made stronger, a thrombus formed unintentionally in the mixed liquid comes into contact with the wall surface of main body portion 10 by centrifugal force. At this time, the thrombus moves toward the outer circumferential side with respect to a filter 71 and a central portion of the filter is not clogged with the thrombus because the filter has a cylindrical shape. The mixed liquid passes through conical filter 71, and then, is introduced into mixed liquid line 70. In the case of the pre-dilution method, mixed liquid line 70 is connected to a dialyzer inlet port. In the case of the post-dilution method, the blood in mixed liquid line 70 is introduced into the patient. The shape of filter 71 is not necessarily limited to the conical shape, and may be a pyramid shape or a so-called frustum shape formed by cutting off a tip of a cone. Alternatively, filter 71 may be a mesh sheet folded in two. In addition, it is preferable that a wall member (obstacle) blocking the flow of the mixed liquid transversely is not provided in main body portion 10.

Particularly when an obstacle blocking the flow along the side surface of the housing is provided on the inner surface of main body portion 10 near port 62, the flow of the blood is transversely blocked, which may cause a turbulent flow of the blood and local staying of the blood. It is desirable that an obstacle blocking a vortex flow is not provided in main body portion 10 at the position of the same height as port 62. With such a configuration, a turbulent flow can be prevented. Particularly, in order to cleanly generate a vortex flow from the side surface to the central portion of the housing, it is preferable that only a space is provided in main body portion 10 at the position of the same height as port 62. In addition, the inner surface of main body portion 10 near port 52 is preferably formed to have the same shape of substantially the same dimension as those of the inner surface of main body portion 10 near port 62, and is also preferably formed to generate a vortex flow in the same rotation direction as that of the blood. With such a configuration, the mixing property can be enhanced. In addition, it is preferable that an obstacle blocking the flow is not provided in main body portion 10 near port 52.

An orifice 32 as a slit is provided in upper space 11. Orifice 32 is desirably provided at a position higher than second inlet 160 serving as an inlet of the blood. In the present embodiment, orifice 32 is provided at a position higher than first inlet 150 serving as an inlet of the dialysate. Since orifice 32 is provided, the medical agent in medical agent injection line 30 is introduced into main body portion 10 through orifice 32.

Since orifice 32 is provided, the dripping height of the medical agent can be lowered. When a liquid level 11a of the mixed liquid becomes abnormally high, the mixed liquid can also be discharged from orifice 32.

The presence of orifice 32 may cause bubbling near orifice 32. Therefore, orifice 32 may be eliminated.

A height H in Fig. 6 refers to a distance from the center of groove 51 to the upper surface of main body portion 10. A height X refers to a distance from the center of groove 61 to the center of groove 51. A height Y refers to a height from the bottom surface of mixing chamber 15 to the center of groove 61.

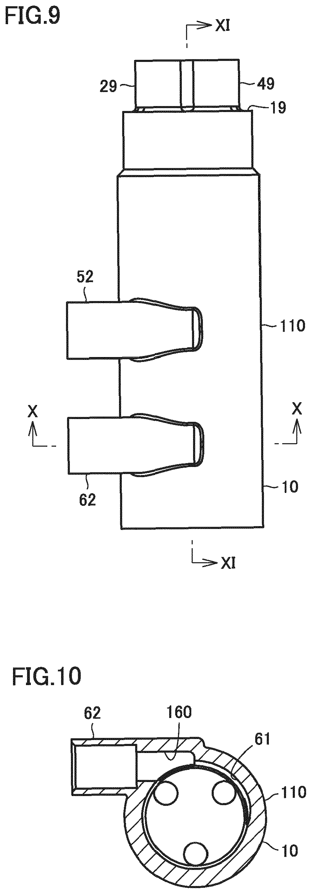

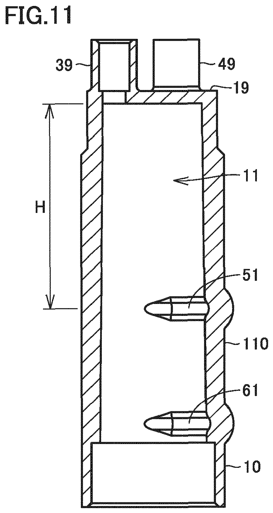

Referring to Figs. 9 to 11, the inner circumferential surface of main body portion 10 has a circular cylindrical shape, and the first and second ports extend in the direction of the tangent to the circular cylinder (i.e., the first and second ports are provided on the side surface side, not at the center of main body portion 10). Therefore, the introduced liquid flows in a swirling manner. The direction of extension of the ports may be a direction substantially orthogonal to the axial direction of main body portion 10, or may be a diagonal direction.

As one example of the configuration having the inlets on the side surface, it is also conceivable to form a port into an L shape and provide the port along the longitudinal direction, and change the flow direction at the L-shaped port in main body portion 10 (US Patent No. 5503801 and European Patent Publication No. 0728509). However, the L-shaped port weakens the momentum of the flow when the liquid hits the lower wall, and thus, the flow velocity becomes lower.

In the mixing apparatus according to the embodiment, the flow velocity of the liquids flowing in from ports 52 and 62 is high, and thus, the flow rate is high. Furthermore, a vortex flow is also likely to occur. When the vortex flow occurs, the time to when the fluid drops onto the bottom portion of main body portion 10 becomes longer. When the time becomes longer, an opportunity to mix a plurality of liquids increases, and thus, the mixing property becomes better.

When the rotation speed of the vortex flow increases, an opportunity to mix with the blood increases, and thus, the mixing property becomes better. Furthermore, as to port 52 serving as the first port, when the flow rate is high, the concentrated blood is less likely to come into contact with the air. Furthermore, at port 62 serving as the second port, the liquid never comes into collision with the wall, and thus, the risk of damaging the blood cells is low.

If port 52 extends diagonally upward, the dialysate layer becomes thick. The excessively thick dialysate layer may worsen the mixing property.

If port 52 extends diagonally downward, the velocity of the liquid does not decrease and the liquid reaches the bottom of main body portion 10 immediately. As a result, the mixing property becomes worse than that of the embodiment.

If port 62 serving as a blood port extends diagonally upward, the blood is likely to come into contact with the air.

If port 62 extends diagonally downward, the number of rotation (rotation speed) of the blood in main body portion 10 is small, and thus, the mixing property is worse than that of the embodiment.

Main body portion 10 has an upper member 110 and a lower member 120. Lower member 120 is inserted into upper member 110. A reason why main body portion 10 is divided into upper member 110 and lower member 120 is to avoid the complication of a molding method. If port 52 is provided on upper member 110 and port 62 is provided on lower member 120, upper member 110 and lower member 120 both have a complicated configuration. As a result, a die is complicated. In the above-described embodiment, ports 52 and 62 are provided on upper member 110 and upper member 110 has a complicated shape, while lower member 120 has a simple shape. As a result, the productivity is increased.

Furthermore, no port is provided on lower member 120 and the inner circumferential surface of lower member 120 is formed to have a circular cylindrical shape. Thus, a laminar vortex flow is likely to occur in lower member 120. As a result, the blood and the displacement liquid are mixed more easily in lower member 120 than the case in which a turbulent flow occurs in lower member 120.

At a contact portion of upper member 110 and lower member 120, lower member 120 is located on the inner side and upper member 110 is located on the outer side. The inner circumferential surface of lower member 120 has a circular cylindrical shape. With such a configuration, the area of contact between the flow of the mixed liquid and lower member 120 increases and a vortex flow (swirling flow) is likely to occur in lower member 120.

Dimension Y in Fig. 6 is preferably not smaller than 40 mm. Dimension Y of not smaller than 40 mm is enough for the distance for mixing, and thus, the mixing property becomes better.

In addition, grooves 51 and 61 extend along the direction of the tangent to the circular cylinder of main body portion 10. As a result, the direction of the liquids flowing through grooves 51 and 61 is gradually changed with decreasing speed, and the liquids smoothly mix with the liquid in main body portion 10. Therefore, bubbling is less likely to occur. In addition, since the liquids flowing through grooves 51 and 61 swirl on the inner circumferential surface of the circular cylinder, vortexes of the liquids are likely to occur in main body portion 10. In addition, when the displacement liquid introduced from the side surface of main body portion 10 forms a swirling flow, the blood and the displacement liquid can be mixed more easily than the conventional art. Specifically, conventionally, the displacement liquid has been dropped from above the liquid level, and thus, the displacement liquid has not dropped uniformly, i.e., the displacement liquid has dropped quickly in some places or has been pushed back and dropped only slowly in some places. However, such a situation can be prevented. In addition, since a moving distance of the displacement liquid in main body portion 10 becomes longer, the displacement liquid is likely to be mixed with the blood. Furthermore, when the liquids having flown through grooves 51 and 61 form vortexes in the same rotation direction, the blood and the displacement liquid gradually drop in a uniform manner while rotating in the same direction. Therefore, the displacement liquid and the blood are mixed more uniformly. In addition, a turbulent flow can become less likely to occur and bubbling can become less likely to occur. The vortexes in the same rotation direction generated by the first port and the second port do not necessarily need to be vortexes that are different in component and bubble between the central portion and the peripheral portion due to specific gravity, and the strength of the vortexes does not matter.

The blood and the displacement liquid can be mixed in main body portion 10 to obtain a uniform mixed liquid.

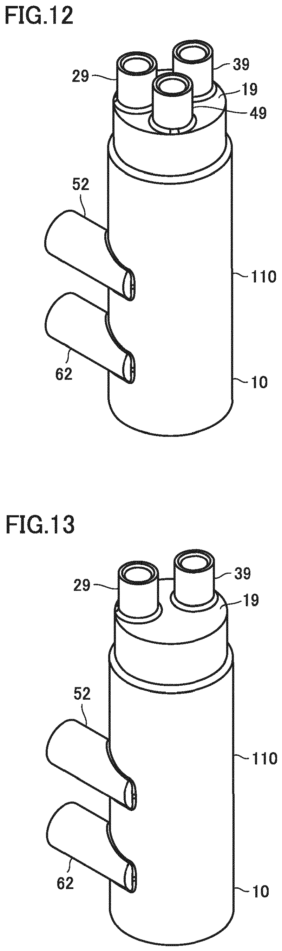

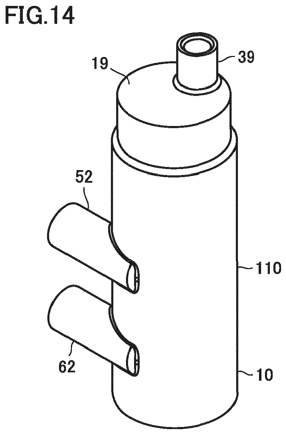

Referring to Figs. 12 to 14, three ports 29, 39 and 49 are formed on an upper surface 19. Three ports 29, 39 and 49 are equally spaced apart from one another.

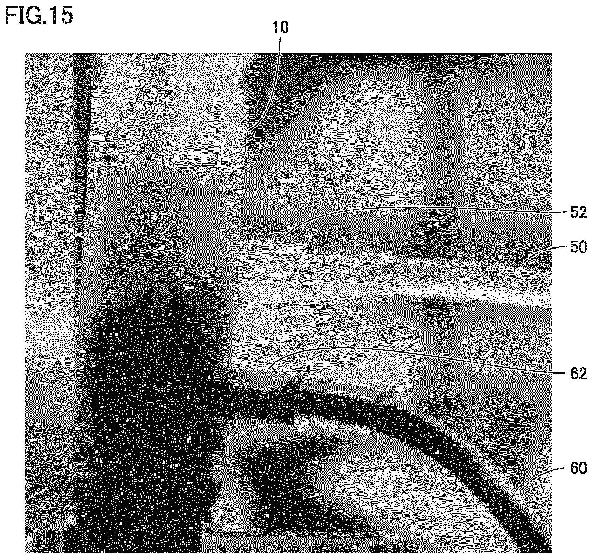

Referring to Fig. 15, the blood is introduced into main body portion 10 from port 62 located on the lower side. The displacement liquid is introduced into main body portion 10 from port 52 located on the upper side. Two types of liquids swirl in main body portion 10. As a result, the displacement liquid and the blood are uniformly mixed.

The replacement liquid (displacement liquid) is introduced into main body portion 10 at the timing of rotating a replacement liquid pump simultaneously with the blood pump after sticking needles provided at opposing ends of the blood circuit into the patient. The replacement liquid pump is continuously driven until the completion of dialysis. It is preferable that formation of a layer of the replacement liquid continues until the completion of dialysis.

In mixing chamber 15, the first port is formed to extend from the side surface portion of the chamber in the direction of the tangent to the circular cylindrical shape, and the second port is formed to extend from the side surface portion of the chamber in the direction of the tangent to the circular cylindrical shape such that a vortex flow occurs in the same rotation direction as that of a vortex flow generated by the first port. As a result, the displacement liquid and the blood are uniformly mixed and bubbling is less likely to occur in the process of mixing. Furthermore, the first port is provided above the second port, and thus, a layer of water vortex is likely to be formed above the blood. As a result, formation of thrombus caused by contact between the blood and the air can become less likely. In addition, in the Examples, the first port and the second port extend in the same direction. With such a configuration, reduction in size of the chamber and prevention of a kink in the lines connected to the ports can be achieved. The first port and the second port may be provided such that a vortex flow caused by the direction of the first port is opposite to a vortex flow caused by the direction of the second port. Alternatively, the first port and the second port may be arranged in the central portion of the chamber. In this case, a turbulent flow is more likely to occur and a vortex flow is less likely to occur. As a result, bubbling is more likely to occur as compared with the case in which the rotation direction of the vortex flow generated by the first port is the same as the rotation direction of the vortex flow generated by the second port.

The suitable volume of upper space 11 located above ports 52 and 62 serving as the inlets is not smaller than 0.5 cm³. The blood pump in Fig. 1 is a roller pump. In the roller pump, backflow (pulsating) occurs. Therefore, the liquid level in mixing chamber 15 goes up and down. At this time, when the liquid level of the blood in main body portion 10 goes below an upper end of port 52, the air enters displacement liquid line 50 and bubbling occurs. In order to effectively suppress bubbling, the volume of the upper space is preferably not smaller than 0.5 cm³.

When the flow rates of the displacement liquid and the blood are set to be very high, the internal pressure and the liquid level in mixing chamber 15 may increase and the blood may flow into pressure monitoring line 20. As a result, there arises a problem of occurrence of bubbling in main body portion 10 or formation of thrombus in pressure monitoring line 20 or in the upper portion of main body portion 10. In order to suppress the occurrence of such a problem, it is preferable that upper space 11 has a sufficient volume. The volume of upper space 11 is preferably not smaller than 3 cm³. If the volume of upper space 11 is larger than 9 cm³, mixing chamber 15 increases in size. Therefore, the volume of upper space 11 is preferably 3 to 9 cm³.

Inner diameter D is preferably 10 to 35 mm. If inner diameter D is smaller than 10 mm, inner diameter D is too small and the air trap effect is reduced. If inner diameter D exceeds 35 mm, a priming volume is too large.

The first port may be an introduction port during priming. When bubbles are formed during priming, the blood comes into contact with the bubbles at the time of replacement of the priming liquid (normal saline) with the blood, which may cause formation of thrombus. However, the present invention can make such a situation less likely to occur.

### (Example 1)

In Example 1 described below, dimensions X and Y shown in Fig. 6 were variously changed and the uniformity of mixing of the water and the blood was examined with simulation.

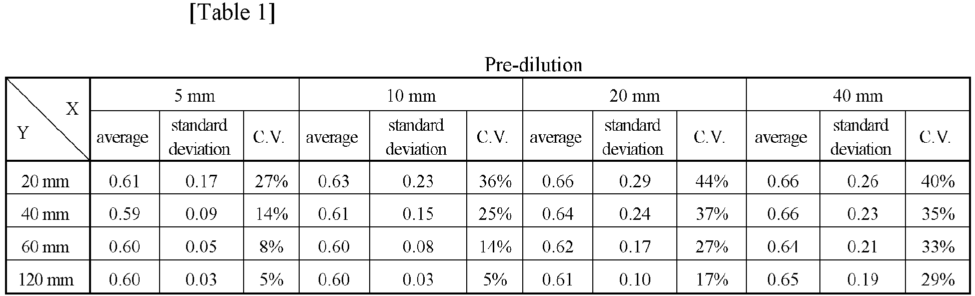

**[Table 2]**

| blood | water | blood ratio |
|---|---|---|
| 250 | 180 | 0.58 |

Table 1 shows a ratio of the blood in the mixed liquid when X and Y in Fig. 6 are changed in the case of pre-dilution. Specifically, "average" in Table 1 represents an average ratio of the blood in the mixed liquid. The standard deviation is a value indicating variability of a plurality of samples used to obtain the average. As the value of the standard deviation becomes larger, the variability becomes greater. The ratio between the water and the blood was calculated immediately after the water and the blood passed through filter 71 and entered mixed liquid line 70.

H was set at 30 mm and D was set at 15 mm. Table 2 shows a flow rate ratio between the blood and the water introduced into main body portion 10. The unit of the flow rate is cm³/min.

According to Tables 1 and 2, the ratio of the blood in the mixed liquid is lower than that of Example 2 described below.

It can be seen from Table 1 that the mixed liquid has a uniform composition.

### (Example 2)

In Example 2 described below, dimensions X and Y shown in Fig. 6 were variously changed and the uniformity of mixing of the water and the blood was examined with simulation.

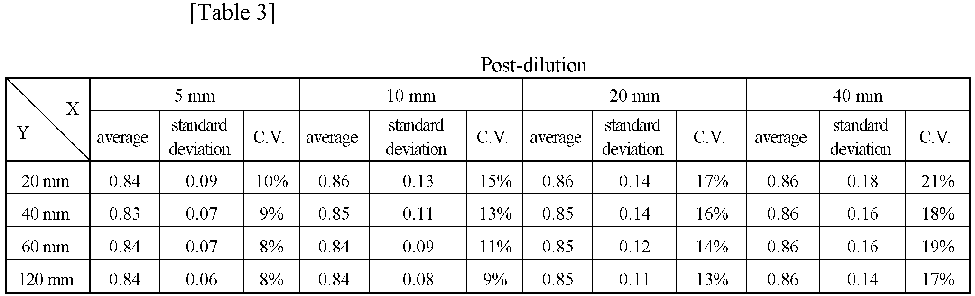

**[Table 4]**

| blood | water | blood ratio |
|---|---|---|
| 250 | 60 | 0.81 |

Table 3 shows a ratio of the blood in the mixed liquid when X and Y in Fig. 6 are changed in the case of post-dilution. Specifically, "average" in Table 3 represents an average ratio of the blood in the mixed liquid. The ratio between the water and the blood was calculated immediately after the water and the blood passed through filter 71 and entered mixed liquid line 70.

H was set at 30 mm and D was set at 15 mm. Table 4 shows a flow rate ratio between the blood and the water introduced into main body portion 10. The unit of the flow rate is cm³/min.

According to Tables 3 and 4, the ratio of the blood in the mixed liquid is higher than that of Example 1 described above. Therefore, the mixing conditions in Example 2 are the same as those of the post-dilution.

It can be seen from Table 3 that the mixed liquid has a uniform composition.

### (Example 3)

In Example 3 described below, dimensions X and Y shown in Fig. 6 were variously changed and the uniformity of mixing of the water and the blood was examined with simulation.

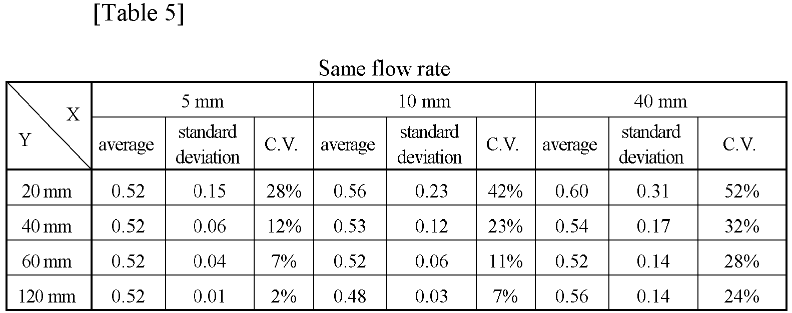

**[Table 6]**

| blood | water | blood ratio |
|---|---|---|
| 250 | 250 | 0.50 |

Table 5 shows a ratio of the blood in the mixed liquid when X and Y in Fig. 6 are changed in the case where a flow rate of the blood and a flow rate of the displacement liquid are the same. Specifically, "average" in Table 5 represents an average ratio of the blood in the mixed liquid. The standard deviation is a value indicating variability of a plurality of samples used to obtain the average. As the value of the standard deviation becomes larger, the variability becomes greater. The ratio between the water and the blood was calculated at a position immediately after the water and the blood passed through filter 71 and entered mixed liquid line 70.

H was set at 30 mm and D was set at 15 mm. Table 6 shows a flow rate ratio between the blood and the water introduced into main body portion 10. The unit of the flow rate is cm³/min.

It can be seen from Table 5 that the mixed liquid has a uniform composition.

### (Example 4)

In Example 4 described below, dimensions X and Y shown in Fig. 6 were variously changed and the uniformity of mixing of the water and the blood was examined with simulation.

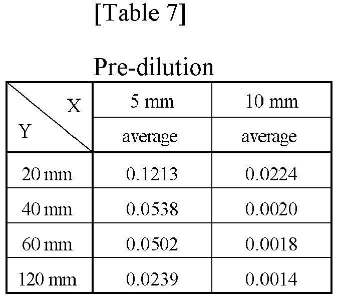

**[Table 8]**

| blood | water | blood ratio |
|---|---|---|
| 250 | 180 | 0.58 |

Table 7 shows a ratio of the blood in the mixed liquid when X and Y in Fig. 6 are changed in the case of pre-dilution. Specifically, "average" in Table 7 represents an average ratio of the blood in the mixed liquid. The ratio between the water and the blood was calculated at a position of liquid level 11a.

H was set at 30 mm and D was set at 15 mm. Table 8 shows a flow rate ratio between the blood and the water introduced into main body portion 10. The unit of the flow rate is cm³/min.

It can be seen from Table 7 that the concentration of the blood may increase at liquid level 11a when X is 5 mm and Y is 20 mm.

### (Example 5)

In Example 5 described below, dimensions X and Y shown in Fig. 6 were variously changed and the uniformity of mixing of the water and the blood was examined with simulation.

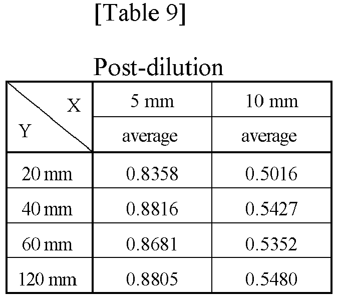

**[Table 10]**

| blood | water | blood ratio |
|---|---|---|
| 250 | 60 | 0.81 |

Table 9 shows a ratio of the blood in the mixed liquid when X and Y in Fig. 6 are changed in the case of post-dilution. Specifically, "average" in Table 9 represents an average ratio of the blood in the mixed liquid. The ratio between the water and the blood was calculated at a position of liquid level 11a.

H was set at 30 mm and D was set at 15 mm. Table 10 shows a flow rate ratio between the blood and the water introduced into main body portion 10. The unit of the flow rate is cm³/min.

It can be seen from Table 9 that the concentration of the blood may increase at liquid level 11a when X is 5 mm.

### (Example 6)

In Example 6 described below, dimensions X and Y shown in Fig. 6 were variously changed and the uniformity of mixing of the water and the blood was examined with simulation.

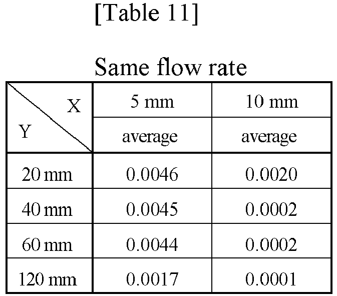

**[Table 12]**

| blood | water | blood ratio |
|---|---|---|
| 250 | 250 | 0.50 |

Table 11 shows a ratio of the blood in the mixed liquid when X and Y in Fig. 6 are changed in the case where a flow rate of the blood and a flow rate of the displacement liquid are the same. Specifically, "average" in Table 11 represents an average ratio of the blood in the mixed liquid. The ratio between the water and the blood was calculated at a position of liquid level 11a.

H was set at 30 mm and D was set at 15 mm. Table 12 shows a flow rate ratio between the blood and the water introduced into main body portion 10. The unit of the flow rate is cm³/min.

It can be seen from Table 11 that the concentration of the blood is low at liquid level 11a.

These embodiments and examples can be variously changed. First, as to the height of liquid level 11a, an amount of the liquid flowing into mixing chamber 15 and an amount of the liquid flowing out from mixing chamber 15 may be controlled in order to adjust the height of liquid level 11a.

Furthermore, the example of using mixing chamber 15 in online HDF has been described. However, mixing chamber 15 may be used in offline HDF. In the case of offline HDF, a displacement liquid (dialysate) in a bag and the blood are mixed to form a mixed liquid. Specifically, displacement liquid line 50 is connected to the bag filled with the displacement liquid.

Furthermore, mixing chamber 15 described above can be used not only in HDF but also in blood dialysis (HD) and blood filtration (HF).

It should be understood that the embodiments disclosed herein are illustrative and not limitative in any respect. The scope of the present invention is defined by the terms of the claims, rather than the description above, and is intended to include any modifications within the scope and meaning equivalent to the terms of the claims.

### INDUSTRIAL APPLICABILITY

The present invention can be used in the field of a chamber that mixes blood and a displacement liquid, and particularly a chamber used together with a dialysis apparatus.

### REFERENCE SIGNS LIST

1 blood introduction port; 2 blood removal pressure measurement site; 3, 6, 112 pressure transducer; 4 blood pump; 5 pressure measurement site; 7a dialyzer blood introduction port; 7b dialyzer blood delivery port; 8 dialyzer; 9a dialysate delivery port; 9b dialysate introduction port; 10 main body portion; 11 upper space; 11a liquid level; 15 mixing chamber; 19 upper surface; 20 pressure measurement line; 21 transducer protection filter; 29, 39, 49, 52, 62, 72 port; 30 medical agent injection line; 31, 41 clip; 32 orifice; 40 liquid level adjustment line; 50 displacement liquid line; 51, 61 groove; 60 blood line; 70 mixed liquid line; 71 filter; 100 dialysis apparatus main body; 110 upper member; 111 venous pressure measurement site; 113 blood delivery port; 120 lower member; 150 first inlet; 160 second inlet.

## Claims

1. A mixing chamber (15) provided on a blood circuit and including a main body portion (10) having a cylindrical shape,
the main body portion (10) having, on a side surface of the cylindrical shape, a first inlet (150) through which a liquid is introduced into a space of the cylindrical shape and a second inlet through (160) which blood is introduced,
a space being provided to extend from the first inlet (150) to a highest portion of the main body portion (10)
the mixing chamber (15) being configured such that the liquid and the blood introduced from the first and the second inlets (150, 160) into the main body portion (10) move toward a direction displaced from a center of the main body portion (10),
wherein
the inner circumferential surface of the main body portion (10) guides, in a swirling manner, the liquid flowing from the first inlet (150) into the main body portion (10),
and wherein
the inner circumferential surface of the main body portion (10) guides, in a swirling manner, the blood flowing from the second inlet (160) into the main body portion (10), wherein the liquid and the blood introduced from the first inlet (150) and the second inlet (160) are guided in one rotation direction along the surface of the main body portion (10), the first inlet (150) being provided above the second inlet (160).

2. The mixing chamber according to claim 1, wherein
a curved surface portion configured to guide the liquid in a rotation direction is formed on an inner surface of the main body portion (10) in a flow direction of the liquid introduced from the first inlet (150) into the main body portion (10),

3. The mixing chamber (15) according to claims 1or 2, wherein
the inner circumferential surface of the main body portion (10) from the first inlet (150) to the second inlet (160) has a substantially circular cylindrical shape.

4. The mixing chamber (15) according to claim 3, wherein
the inner circumferential surface of the main body portion (10) has a curved surface, and at least one of the first inlet (150) and the second inlet (160) extends along a direction of a tangent to the curved surface.

5. The mixing chamber (15) according to any one of claims 1 to 4, wherein the main body portion (10) extends in a longitudinal direction, the main body portion (10) has a first port (52) extending to be substantially orthogonal to the longitudinal direction, one end of the first port (52) is connected to the first inlet (150), and the other end of the first port (52) is connected to a displacement liquid line (50).

6. The mixing chamber (15) according to claim 5, wherein
the main body portion (10) extends in the longitudinal direction, the main body portion (10) has a second port (62) extending to be substantially orthogonal to the longitudinal direction, one end of the second port (62) is connected to the second inlet (160), and the other end of the second port (62) is connected to a blood line (60).

7. The mixing chamber (15) according to claim 6, wherein
a distance from a bottom of the main body portion (10) to the second port (62) is not shorter than 40 mm.

8. The mixing chamber (15) according to claim 6 or 7, wherein
a distance between the first port (52) and the second port (62) is not shorter than 0.5 cm.

9. The mixing chamber (15) according to any one of claims 6 to 8, wherein
a groove (51, 61) connecting to at least one of the first port (52) and the second port (62) is provided in the side surface of the main body portion (10).

10. The mixing chamber (15) according to any one of claims 1 to 9, wherein a volume of the space is not smaller than 0.5 cm³.

11. The mixing chamber (15) according to claim 9, wherein
a volume of the space is not smaller than 3 to 9 cm³ and an inner diameter of the space is 10 to 35 mm.

12. The mixing chamber (15) according to any one of claims 1 to 11,
wherein
a flow resistance of a mixed liquid is substantially equal at respective height surfaces in the main body portion (10).

13. The mixing chamber (15) according to any one of claims 1 to 12,
wherein
a transversely blocking wall member that interferes with a vortex flow is not provided in the main body portion (10).

14. The mixing chamber (15) according to any one of claims 1 to 13,
wherein
the main body portion (10) includes an upper member (110) that is provided with the first inlet (150) and the second inlet (160), and a circular cylindrical lower member (120) that is not provided with the first inlet (150) and the second inlet (160) and is in contact with the upper member (110).

## Patentansprüche

1. Mischkammer (15), die an einem Blutkreislauf vorgesehen ist und einen Hauptkörperabschnitt (10) mit einer zylindrischen Form aufweist,
wobei der Hauptkörperabschnitt (10) an einer Seitenfläche der zylindrischen Form einen ersten Einlass (150), durch den eine Flüssigkeit in einen Raum der zylindrischen Form eingeführt wird, und einen zweiten Einlass (160), durch den Blut eingeführt wird, aufweist,
einen Raum, der sich vom ersten Einlass (150) bis zu einem höchsten Abschnitt des Hauptkörperabschnitt (10) erstreckt, wobei
die Mischkammer (15) so angepasst ist, dass die Flüssigkeit und das Blut, die von den ersten und zweiten Einlässen (150, 160) in den Hauptkörperabschnitt (10) eingeführt werden, sich in eine Richtung bewegen, die von einer Mitte des Hauptkörperabschnitts (10) versetzt ist,
wobei die innere Umfangsfläche des Hauptkörperabschnitts (10) die vom ersten Einlass (150) in den Hauptkörperteil (10) fließende Flüssigkeit verwirbelnd leitet, und wobei die innere Umfangsfläche des Hauptkörperabschnitts (10) das aus dem zweiten Einlass (160) in den Hauptkörperabschnitt (10) fließende Blut verwirbelnd leitet, wobei die Flüssigkeit das Blut, die aus dem ersten Einlass (150) und dem zweiten Einlass (160) eingeleitet werden, in einer Drehrichtung entlang der Oberfläche des Hauptkörperabschnitts (10) geführt werden, wobei der erste Einlass (150) oberhalb des zweiten Einlasses (160) vorgesehen ist.

2. Mischkammer gemäß Anspruch 1, wobei
ein gekrümmter Oberflächenabschnitt, der so angepasst ist, dass er die Flüssigkeit in einer Rotationsrichtung führt, auf einer inneren Oberfläche des Hauptkörperabschnitts (10) in einer Strömungsrichtung der Flüssigkeit ausgebildet ist, die von dem ersten Einlass (150) in den Hauptkörperabschnitt (10) eingeführt wird.

3. Mischkammer (15) gemäß Anspruch 1 oder 2, wobei
die innere Umfangsfläche des Hauptkörperabschnitts (10) vom ersten Einlass (150) zum zweiten Einlass (160) eine im Wesentlichen kreiszylindrische Form aufweist.

4. Mischkammer (15) gemäß Anspruch 3, wobei
die innere Umfangsfläche des Hauptkörperabschnitts (10) eine gekrümmte Oberfläche aufweist, und mindestens einer von dem ersten Einlass (150) und dem zweiten Einlass (160) sich entlang einer Richtung einer Tangente zu der gekrümmten Oberfläche erstreckt.

5. Mischkammer (15) gemäß einem der Ansprüche 1 bis 4, wobei
der Hauptkörperabschnitt (10) sich in einer Längsrichtung erstreckt, der Hauptkörperabschnitt (10) eine erste Öffnung (52) aufweist, die sich im Wesentlichen orthogonal zu der Längsrichtung erstreckt, ein Ende der ersten Öffnung (52) mit dem ersten Einlass (150) verbunden ist und das andere Ende der ersten Öffnung (52) mit einer Verdrängungsflüssigkeitsleitung (50) verbunden ist.

6. Mischkammer (15) gemäß Anspruch 5, wobei
der Hauptkörperabschnitt (10) sich in der Längsrichtung erstreckt, der Hauptkörperabschnitt (10) eine zweite Öffnung (62) aufweist, die sich im Wesentlichen orthogonal zu der Längsrichtung erstreckt, ein Ende der zweiten Öffnung (62) mit dem zweiten Einlass (160) verbunden ist und das andere Ende der zweiten Öffnung (62) mit einer Blutleitung (60) verbunden ist.

7. Mischkammer (15) gemäß Anspruch 6, wobei
ein Abstand von einem Boden des Hauptkörperabschnitts (10) zu der zweiten Öffnung (62) nicht kürzer als 40 mm ist.

8. Mischkammer (15) gemäß Anspruch 6 oder 7, wobei
ein Abstand zwischen der ersten Öffnung (52) und der zweiten Öffnung (62) nicht kürzer als 0,5 cm ist.

9. Mischkammer (15) gemäß einem der Ansprüche 6 bis 8, wobei
eine Nut (51, 61), die mit mindestens einer der ersten Öffnung (52) und der zweiten Öffnung (62) verbunden ist, in der Seitenfläche des Hauptkörperabschnitts (10) vorgesehen ist.

10. Mischkammer (15) gemäß einem der Ansprüche 1 bis 9, wobei das Volumen des Raums nicht kleiner als 0,5 cm³ ist.

11. Mischkammer (15) gemäß Anspruch 9, wobei
das Volumen des Raums nicht kleiner als 3 bis 9 cm³ ist, und der Innendurchmesser des Raums 10 bis 35 mm beträgt.

12. Mischkammer (15) gemäß einem der Ansprüche 1 bis 11, wobei
ein Strömungswiderstand einer gemischten Flüssigkeit an den jeweiligen Höhenflächen im Hauptkörperabschnitt (10) im Wesentlichen gleich ist.

13. Mischkammer (15) gemäß einem der Ansprüche 1 bis 12, wobei
ein quer blockierendes Wandelement, das eine Wirbelströmung behindert, im Hauptkörperabschnitt (10) nicht vorgesehen ist.

14. Mischkammer (15) gemäß einem der Ansprüche 1 bis 13, wobei
der Hauptkörperabschnitt (10) ein oberes Element (110), das mit dem ersten Einlass (150) und dem zweiten Einlass (160) versehen ist, und ein kreiszylindrisches unteres Element (120) umfasst, das nicht mit dem ersten Einlass (150) und dem zweiten Einlass (160) versehen ist und in Kontakt mit dem oberen Element (110) steht.

## Revendications

1. Chambre de mélange (15) fournie sur un circuit de sang incluant une partie de corps principal (10) ayant une forme cylindrique,
la partie de corps principal (10) ayant, sur une surface latérale de la forme cylindrique, un premier orifice d'admission (150) à travers lequel un liquide est introduit dans un espace de la forme cylindrique et un second orifice d'admission (160) à travers lequel du sang est introduit,
un espace étant fourni pour s'étendre à partir du premier orifice d'admission (150) jusqu'à une partie la plus élevée de la partie de corps principal (10)
la chambre de mélange (15) étant configurée de manière à ce que le liquide et le sang introduits par les premier et second orifices d'admission (150, 160) dans la partie de corps principal (10) se déplacent vers une direction déplacée d'un centre de la partie de corps principal (10),
dans laquelle
la surface circonférentielle interne de la partie de corps principal (10) guide, en définissant un tourbillon, le liquide s'écoulant depuis le premier orifice d'admission (150) dans la partie de corps principal (10),
et dans laquelle
la surface circonférentielle interne de la partie de corps principal (10) guide, en définissant un tourbillon, le sang s'écoulant du second orifice d'admission (160) dans la partie de corps principal (10), dans laquelle le liquide et le sang introduits par le premier orifice d'admission (150) et le second orifice d'admission (160) sont guidés dans une direction de rotation le long de la surface de la partie de corps principal (10), le premier orifice d'admission (150) étant fourni par-dessus le second orifice d'admission (160).

2. Chambre de mélange selon la revendication 1, dans laquelle
une partie surface incurvée configurée pour guider le liquide dans une direction de rotation est formée sur une surface interne de la partie de corps principal (10) dans une direction d'écoulement du liquide introduit par le premier orifice d'admission (150) dans la partie de corps principal (10).

3. Chambre de mélange (15) selon les revendications 1 ou 2, dans laquelle
la surface circonférentielle interne de la partie de corps principal (10) du premier orifice d'admission (150) au second orifice d'admission (160) a une forme cylindrique sensiblement circulaire.

4. Chambre de mélange (15) selon la revendication 3, dans laquelle
la surface circonférentielle interne de la partie de corps principal (10) a une surface incurvée, et au moins l'un du premier orifice d'admission (150) et du second orifice d'admission (160) s'étend dans une direction d'une tangente à la surface incurvée.

5. Chambre de mélange (15) selon l'une quelconque des revendications 1 à 4, dans laquelle
la partie de corps principal (10) s'étend dans une direction longitudinale, la partie de corps principal (10) a un premier port (52) s'étendant pour être sensiblement orthogonal à la direction longitudinale, une extrémité du premier port (52) est raccordée au premier orifice d'admission (150), et l'autre extrémité du premier port (52) est raccordée à une ligne de déplacement de liquide (50).

6. Chambre de mélange (15) selon la revendication 5, dans laquelle
la partie de corps principal (10) s'étend dans une direction longitudinale, la partie de corps principal (10) a un second port (62) s'étendant pour être sensiblement orthogonal à la direction longitudinale, une extrémité du second port (62) est raccordée au second orifice d'admission (160), et l'autre extrémité du second port (62) est raccordée à une ligne de sang (60) .

7. Chambre de mélange (15) selon la revendication 6, dans laquelle
une distance à partir d'une partie inférieure de la partie de corps principal (10) jusqu'au second port (62) n'est pas inférieure à 40 mm.

8. Chambre de mélange (15) selon la revendication 6 ou 7, dans laquelle
une distance entre le premier port (52) et le second port (62) n'est pas inférieure à 0,5 cm.

9. Chambre de mélange (15) selon l'une quelconque des revendications 6 à 8, dans laquelle
une rainure (51, 61) se raccordant à au moins l'un du premier port (52) et du second port (62) est fournie dans la surface latérale de la partie de corps principal (10).

10. Chambre de mélange (15) selon l'une quelconque des revendications 1 à 9, dans laquelle
un volume de l'espace n'est pas inférieur à 0, 5 cm³.

11. Chambre de mélange (15) selon la revendication 9, dans laquelle
un volume de l'espace n'est pas inférieur à 3 à 9 cm³ et un diamètre interne de l'espace est de 10 à 35 mm.

12. Chambre de mélange (15) selon l'une quelconque des revendications 1 à 11,
dans laquelle
une résistance à l'écoulement d'un liquide mixte est sensiblement égale au niveau de surfaces de hauteurs respectives dans la partie de corps principal (10) .

13. Chambre de mélange (15) selon l'une quelconque des revendications 1 à 12,
dans laquelle
un organe de paroi de blocage transversal qui interfère avec un écoulement de vortex n'est pas fourni dans la partie de corps principal (10).

14. Chambre de mélange (15) selon l'une quelconque des revendications 1 à 13, dans laquelle
la partie de corps principal (10) inclut un organe supérieur (110) qui est doté du premier orifice d'entrée (150) et du second orifice d'entrée (160), et un organe inférieur cylindrique circulaire (120) qui n'est pas doté du premier orifice d'entrée (150) ni du second orifice d'entrée (160) et est en contact avec l'organe supérieur (110).
